# EUROPEAN PATENT SPECIFICATION

(11) **EP 2 010 221 B1**
(45) Date of publication and mention of the grant of the patent: **29.02.2012**
(21) Application number: 07734878.7
(22) Date of filing: 24.04.2007
(51) Int. Cl.: A61K 47/48, A61P 35/00, A61P 33/00

(54) **ANTIBODY-RNASE-CONJUGATE**
ANTIKÖRPER-RNASE-KONJUGAT
CONJUGUÉ ANTICORPS-RNASE

(30) Priority: 21.04.2006 EP 06112942
(43) Date of publication of application: 07.01.2009
(73) Proprietor: mAB-Factory GmbH, 38108 Braunschweig (DE)
(72) Inventor: DUBEL, Stefan, 38106 Braunschweig (DE); SCHIRRMANN, Thomas, 10997 Berlin (DE); MENZEL, Christian, 64319 Pfungstadt (DE); JOSTOCK, Thomas, 38530 Didderse (DE)
(74) Representative: Taruttis, Stefan Georg
(86) International application number: PCT/IB2007/001687
(87) International publication number: WO 2007/122511

(56) References cited:
- WO-A-01/19846
- WO-A-02/069886
- WO-A-03/006509
- WO-A-03/051926
- WO-A-2005/080586
- US-A- 5 892 019
- US-A1- 2002 192 223
- US-A1- 2003 152 568
- US-A1- 2005 266 010
- US-B1- 6 410 319
- STÖCKER M ET AL: "Secretion of functional anti-CD30-angiogenin immunotoxins into the supernatant of transfected 293T-cells" PROTEIN EXPRESSION AND PURIFICATION, ACADEMIC PRESS, US, vol. 28, no. 2, April 2003 (2003-04), pages 211-219, XP002302002 ISSN: 1046-5928
- XU X ET AL: "Targeting and therapy of carcinoembryonic antigen-expressing tumors in transgenic mice with an antibody-interleukin 2 fusion protein" CANCER RESEARCH, AMERICAN ASSOCIATION FOR CANCER RESEARCH, BALTIMORE, MD, US, vol. 60, no. 16, 15 August 2000 (2000-08-15), pages 4475-4484, XP002337817 ISSN: 0008-5472
- JENSEN M ET AL: "CD20 is a molecular target for scFvFc:zeta receptor redirected T cells: implications for cellular immunotherapy of CD20+ malignancy" BIOLOGY OF BLOOD AND MARROW TRANSPLANTATION, KLUGE CARDEN JENNINGS PUBLISHING, CHARLOTTESVILLE, VA, US, vol. 4, no. 2, 1998, pages 75-83, XP000910525 ISSN: 1083-8791
- DE LORENZO C ET AL: "A new RNase-based immunoconjugate selectively cytotoxic for ErbB2-overexpressing cells" FEBS LETTERS, ELSEVIER, AMSTERDAM, NL, vol. 516, no. 1-3, 10 April 2002 (2002-04-10), pages 208-212, XP004349079 ISSN: 0014-5793
- RYBAK S M ET AL: "HUMANIZATION OF IMMUNOTOXINS" PROCEEDINGS OF THE NATIONAL ACADEMY OF SCIENCES OF USA, NATIONAL ACADEMY OF SCIENCE, WASHINGTON, DC, US, vol. 89, no. 8, 15 April 1992 (1992-04-15), pages 3165-3169, XP000266946 ISSN: 0027-8424
- ZEWE M ET AL: "Cloning and cytotoxicity of a human pancreatic RNase immunofusion" IMMUNOTECHNOLOGY, ELSEVIER SCIENCE PUBLISHERS BV, NL, vol. 3, no. 2, June 1997 (1997-06), pages 127-136, XP004126675 ISSN: 1380-2933 cited in the application
- NEWTON D L ET AL: "ANGIOGENIN SINGLE-CHAIN IMMUNOFUSIONS : INFLUENCE OF PEPTIDE LINKERS AND SPACERS BETWEEN FUSION PROTEIN DOMAINS" BIOCHEMISTRY, AMERICAN CHEMICAL SOCIETY. EASTON, PA, US, vol. 35, no. 2, 16 January 1996 (1996-01-16), pages 545-553, XP000647484 ISSN: 0006-2960
- NEWTON D L ET AL: "EXPRESSION AND CHARACTERIZATION OF RECOMBINANT HUMAN EOSINOPHIL- DERIVED NEROTOXIN AND EOSINOPHIL-DERIVED NEUROTOXIN-ANTI- TRANSFERRIN RECEPTOR SFV" JOURNAL OF BIOLOGICAL CHEMISTRY, AMERICAN SOCIETY OF BIOLOCHEMICAL BIOLOGISTS, BIRMINGHAM,, US, vol. 269, no. 43, 28 October 1994 (1994-10-28), pages 26739-26745, XP000647481 ISSN: 0021-9258
- KRAUSS JÜRGEN ET AL: "Targeting malignant B-cell lymphoma with a humanized anti-CD22 scFv-angiogenin immunoenzyme." BRITISH JOURNAL OF HAEMATOLOGY MAR 2005, vol. 128, no. 5, March 2005 (2005-03), pages 602-609, XP002463022 ISSN: 0007-1048
- RYBAK S M ET AL: "Natural and engineered cytotoxic ribonucleases: therapeutic potential." EXPERIMENTAL CELL RESEARCH 15 DEC 1999, vol. 253, no. 2, 15 December 1999 (1999-12-15), pages 325-335, XP002463023 ISSN: 0014-4827
- KENANOVA VANIA ET AL: "Tailoring the pharmacokinetics and positron emission tomography imaging properties of anti-carcinoembryonic antigen single-chain Fv-Fc antibody fragments" CANCER RESEARCH, AMERICAN ASSOCIATION FOR CANCER RESEARCH, BALTIMORE, MD, vol. 65, no. 2, 15 January 2005 (2005-01-15), pages 622-631, XP009089506 ISSN: 0008-5472
- MCDONAGH CHARLOTTE F ET AL: "Development of antibody fragment auristatim drug conjugates for cancer therapy" PROCEEDINGS OF THE ANNUAL MEETING OF THE AMERICAN ASSOCIATIONFOR CANCER RESEARCH, NEW YORK, NY, vol. 47, 1 April 2006 (2006-04-01), page 281, XP001536929 ISSN: 0197-016X
- WANG BAIYANG ET AL: "Human single-chain Fv immunoconjugates targeted to a melanoma-associated chondroitin sulfate proteoglycan mediate specific lysis of human melanoma cells by natural killer cells and complement" PROCEEDINGS OF THE NATIONAL ACADEMY OF SCIENCES OF USA, NATIONAL ACADEMY OF SCIENCE, WASHINGTON, DC, vol. 96, 16 February 1999 (1999-02-16), pages 1627-1632, XP002143248 ISSN: 0027-8424

## Description

The present invention relates to fusion proteins that are conjugates having an antibody portion and an RNase portion, currently termed antibody-RNase conjugate (Ab-RNase conjugate). Generic Ab-RNase conjugates are known to be effective as antigen-specific cytotoxic agents, e.g. for use in the manufacture of pharmaceutical compositions.

The present invention provides a favourable production method based on a novel antibody-RNase conjugate which is a single chain protein, providing both the specificity of its antibody portion and the RNase activity of its RNase portion, resulting in an antigen specific effectiveness against cells when applied in vivo or in vitro, wherein the RNase portion is effectively cytotoxic in at least a fraction of cells presenting the antigen, e.g. after internalization by endocytosis.

State of the art

To date, known, Ab-RNase conjugates are single chain (sc) Fv - RNase fusion proteins.

Production of these conjugates is by expression in bacterial systems, yielding inclusion bodies. These inclusion bodies are refolded to generate scFv-RNase conjugates having an active conformation.

The British Journal of Haematology (2005) reports on the generation and functional characterisation of a humanized fusion protein comprising a stable humanized single chain Fv (scFv) having the grafted specificity of the anti-CD 22 murine monoclonal antibody RFB4, fused to human ribonuclease angiogenin. The fusion protein was expressed in transiently transfected CHO cells and could be used to efficiently kill CD22+ tumor cells at an IC50 of 56 nmol/L.

Stöcker et al (Protein Expression an purification, Academic Press, 28, 211-219) describe a single chain variable fragment (scFv) - angiogenine conjugate and De Lorenzo et al (FEBS Letters, 208-212 2002)) describe a further scFv-RNase conjugate.

Rybak et al (PNAS, 3165-3169 (1192)) describe F(ab')₂-like antibody-RNase fusions.

WO03/006509 describes a humanized scFv antibody section having specificity to the ErbB2 receptor, and generally mentions fusions thereof with RNase or protease.

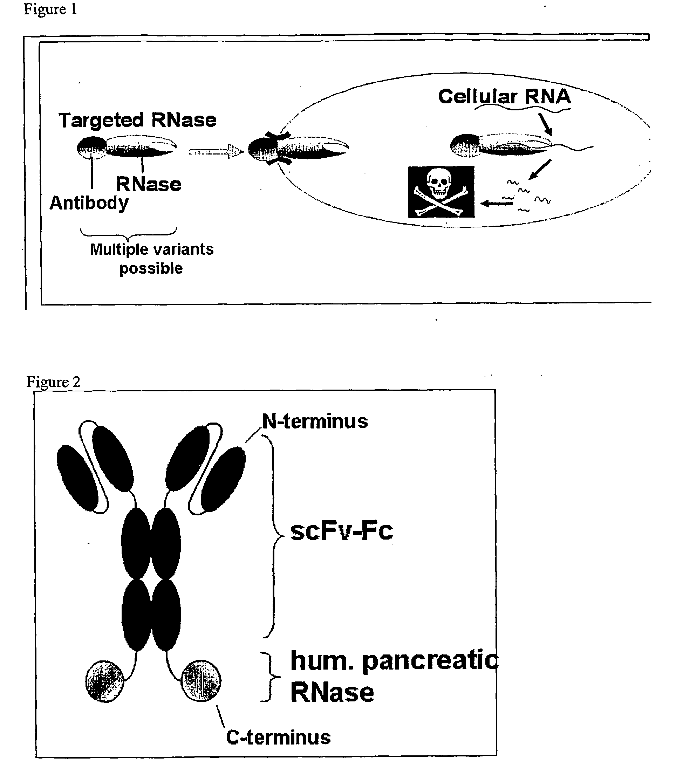

WO 01/019846 describes a method for producing a transgenic fusion protein in the milk of a transgenic animal, wherein the transgenic protein according to Fig. 1 can be an antibody construct according to V_{H}-CH₁-CH₂-CH₃ or V_{H}-C_{L}, or a fusion protein V_{H}-CH₁-shortened CH₂-angiogenine.

Xu et al (Am. Ass. Cancer Research, 4475-4484 (2000)) describe an scFvFc - IL-2 conjugate, having specificity for an extracellular marker named CEA, which is expressed on tumor cells.

Jensen et al (Biology of Blood and Marrow Transpl., 75-73 (1998)) and US 6 410 319 describe a conjugate having an antibody section and a CD4-transmembrane domain coupled to an intracellular signalling domain.

Kenanova et al (Cancer Research, 622 - 631 (2005)) describe a conjugate of antibody domains with RNase, and refer to synthetic antibody formats only.

McDonagh et al (Proceedings of the Annual Meeting of the American Association for Cancer Research, 281 (2006)) refer to synthetic conjugates of synthetic antibody formats with cytotoxic drug molecules.

US 5 892 019 relates to a synthetic antibody without an RNase section conjugated thereto.

DeLorenzo et al. (Cancer Research 64, 4870-4874 (2004)) describe the expression of soluble scFv-RNase fusion protein in the periplasm of E.coli. Further, an scFv RNase conjugate was expressed in mammalian cells, namely in COS7 cells. These known fusion proteins are monovalent with respect to antigen binding and have a small overall size, which is expected to lead to unfavourable pharmacokinetical properties.

A major disadvantage of known Ab-RNase conjugates is their problematic manufacture, often including bacterial expression in the form of inclusion bodies and subsequent folding, having a typical yield of below 10%. Also the production of soluble sc anti-CD 22 Fv-angiogenin conjugates in COS7 cells resulted in an extremely low yield of about 138 ng/mL cell culture supernatant (Krauss et al., British Journal of Haematology, 602-609 (2005)).

Objects of the invention

The present invention seeks to provide cytotoxic antibody-RNase conjugates having a predetermined antigen specificity, which conjugates are single chain proteins that can be produced by expression in eucaryotic cells, e.g. in mammalian cell culture, in higher yields.

Preferably, the antibody-RNase conjugates of the invention have a high target cell specificity in combination with a high cytotoxic activity.

Further, the present invention seeks to provide a production process producing in higher yields a single chain Ab-RNase conjugate having cytotoxic activity towards cells presenting the specific antigen.

Still further, the present invention seeks to provide a pharmaceutical composition comprising the sc Ab-RNase conjugate, utilizing the antigen-specific cytotoxic activity of the conjugate, e.g. for use in the treatment of neoplastic diseases, e.g. against tumors and leukemia.

General description of the invention

The present invention attains the above-mentioned objects by providing an scAb-RNase conjugate which is suitable for being produced by expression in mammalian cell culture, yielding high titers of active conjugate. Further, the scAb-RNase conjugate has the antigen specificity provided by the antibody portion, in combination with the cytotoxic activity provided by the RNase portion.

In detail, the present invention provides scAb-RNase conjugate having the principal structure of scFvFc-RNase. This structure could be shown to allow the effective production of antigen-specific and cytotoxic conjugate protein in cell culture, the conjugate having a high activity with respect to antigen specificity and cytotoxicity.

The conjugates according to the present invention have an arrangement of the RNase portion with its N-terminus linked to the C-terminus of the scFvFc portion. Within the antibody portion the scFv section is N-terminally linked to the Fc section, and the N-terminus of the RNase portion is linked to the C-terminus of the Fc section. This arrangement of the RNase portion to the Ab portion results in the single chain fusion protein to form dimers and, hence, to the advantages of bivalency of the antibody portion as well as a high cytotoxic activity due to the two RNase portions contained in each fusion protein.

The antibody portion is chosen from scFv followed by two constant domains (Fc) of an immunoglobulin, preferably of human origin.

In greater detail, it is preferred that the single chain sequence comprises from N-terminus to C-terminus the following sections:

(an optional) signal peptide - VH - linker - VL - hinge - CH2 - CH3 - linker - RNase.

In this sequence, VH and VL form the Fv section, and CH2 and CH3 form the Fc section, all together forming the scFv-Fc or antibody portion. Within the Fv section, the order of the arrangement of VH and VL can be reversed. Further, the Fv section can be formed by a different single chain antigen-specific amino acid sequence, of which several are known or can be derived from the variable chains of antibodies.

The RNase preferably is human pancreatic RNase.

On the basis of the molecular weight, it is reasonably expected that the fusion protein according to the present invention exhibits a prolonged plasma half life, again increasing its biological activity when applied for medical purposes as part of a pharmaceutical composition. Accordingly, the present invention provides the use of said antibody-RNase conjugates for the manufacture of medicaments for medical use, e.g. for a treatment against tumor cells.

Further, the antigen specificity may be directed against a parasite so that the conjugate can be used as an agent for the treatment of parasitic infections, e.g. against sleeping sickness.

The parasite specificity of the conjugate can be provided by an antibody fragment forming the Fv section, typically a single domain antibody or an scFv antibody. It is preferred that the antibody is specific for and binding to antigen internalised by the parasite, e.g. Trypanosoma brucei, the parasite causing sleeping sickness. One example is specificity towards the transferrin-receptor or the La protein. In conjugates of the invention, the Fv section providing the antigen specificity will be fused to the N-terminus of the Fc-RNase portion, e.g. identical to the one described in Example 1. After specific uptake of the conjugate by the parasite from the infected patient's serum, the parasites are impaired, preferably killed by the RNase activity of the conjugate, independent from their VSG serotype.

As a specific advantage, the sc Ab-RNase conjugates of the invention provide for antigen-specific targeting of the conjugate to target cells, followed by internalization and translocation to the cytoplasm, resulting in cytotoxic effects.

Specifically advantageous of conjugates according to the invention is that all portions correspond to or are of human origin, possibly except for linker portions between the antibody and/or RNase portions, resulting in a low level of immunogenicity. Further, the conjugates according to the present invention allow for high yields in production, e.g. by expression in cell culture, e.g. mammalian cells, including possible post-transcriptional modifications. In accordance with the production of the conjugates in their active, e.g. natural conformation by the expression system, no refolding processes or chemical linkage is necessary.

In accordance with the human origin of antibody and/or RNase portions of the conjugates according to the invention, no serum toxicity is obtained while still providing for high cytotoxicity to target cells.

The RNase portion of Ab-RNase conjugates can be selected from the group comprising bovine seminal RNase, angiogenin ribonuclease, and human pancreatic RNase.

Detailed description of the invention

The present invention will now be described with reference to an scFvFc-RNase, wherein the antibody portion has an anti-CD receptor specificity, and wherein the RNase portion comprises at least in portion human pancreatic RNase.

In the following, reference is made to the figures, wherein

• Figure 1 is a schematic representation of the cytotoxic activity of Ab-RNase conjugates,

• Figure 2 is a schematic representation of the principal structure of scFvFc-RNase conjugates according to the present invention,

• Figure 3 shows an SDS-PAGE of purified samples of the anti-CD receptor scFvFc-RNase conjugate of the invention,

• Figure 4 shows the size exclusion chromatogram of the anti-CD receptor scFvFc-RNase conjugate of the invention, including an SDS-PAGE of fractions,

• Figure 5 shows results of an activity assay of anti-CD receptor scFvFc-RNase conjugate according to the invention, using the decrease of the in vitro transcription of luciferase mRNA as an indicator,

• Figure 6 shows the results of an SPR analysis of conjugates according to the invention to their target antigen,

• Figure 7 shows FACS results of the binding conjugates according to the invention to their antigen specific target cells,

• Figure 8 shows microscopic pictures of cells that have been treated with conjugates according to the invention,

• Figure 9 shows the reduction of proliferation of cells carrying the specific target antigen by conjugates according to the present invention, and

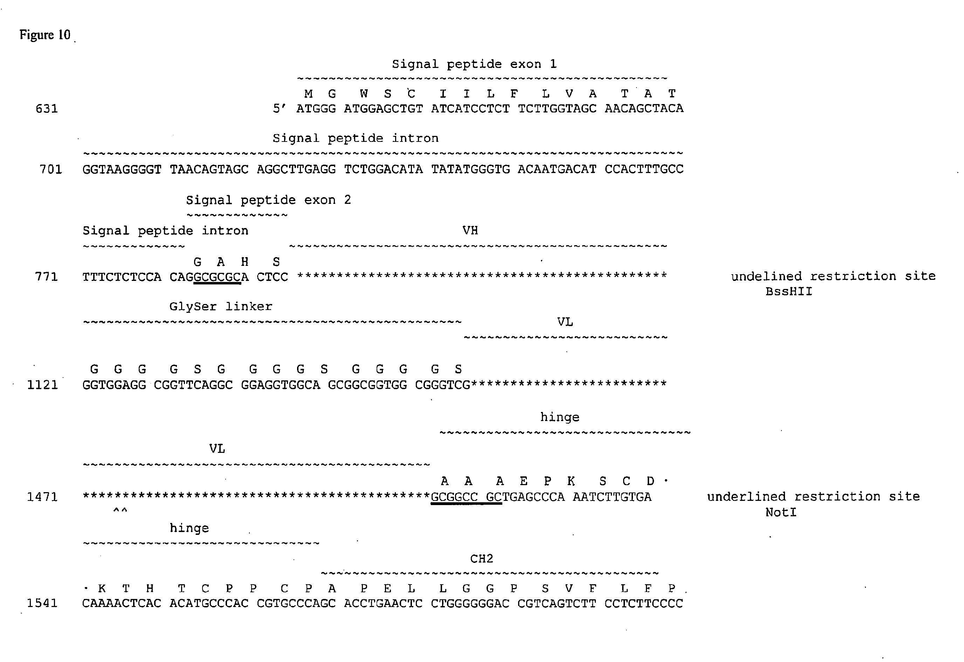

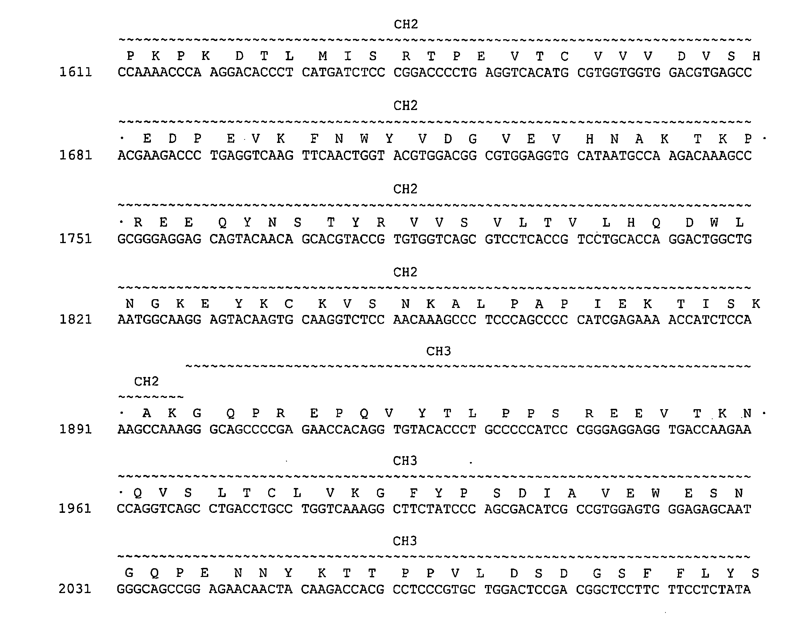

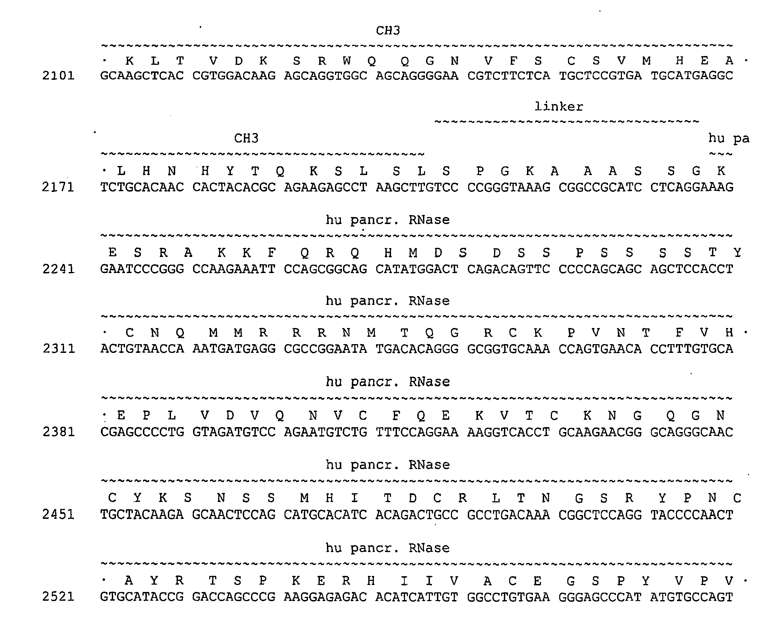

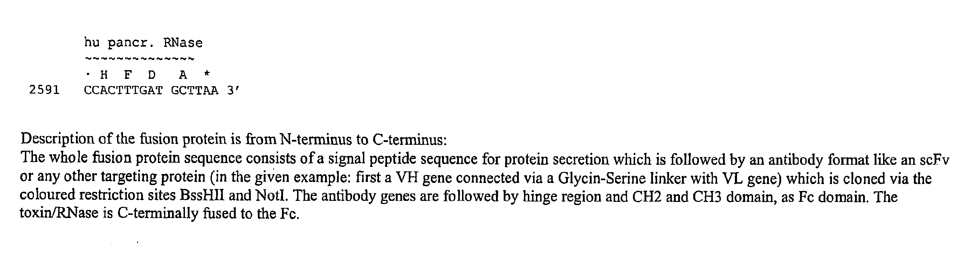

• Figure 10 gives an overview of the sequence elements of one embodiment of anti-CD receptor scFvFc-RNase, wherein nucleotides and amino acids for the antigen-binding section Fv are indicated by stars for VH and VL.

In general, the mechanism for cytotoxic activity of Ab-RNase conjugates is schematically depicted in Figure 1, wherein the conjugate of antibody and RNase is also termed a targeted RNase because of the antigen specificity of the conjugate. In vitro and in vivo activity is triggered by uptake, e.g. endocytosis of the conjugate into the target cell after recognition of an accessible antigen, e.g. a membrane-bound antigen by the Ab portion of the Ab-RNase conjugate. Following uptake into the target cell and translocation into the cytoplasm, the non-specific activity of the RNase portion provides for the cytotoxicity of the conjugate and, as a consequence, leads to a decrease of cell viability, preferably to apoptosis and/or any necrosis of target cells and target tissue.

A schematic representation of the structure of the scFvFc-RNase is given in Figure 2; the DNA sequence of the scFc-RNase portion forming part of the scFvFc-RNase conjugate is enclosed as Seq ID No. 1. Sequence Seq ID No 1 comprises the hinge region to which in 5' a sequence providing antigen specificity, e.g. an Fv section encoding sequence is to be attached at nucleotides 1 to 54, an CH2 domain at nucleotides 55 to 385, a CH3 domain at nucleotides 386 to 693, a linker at nucleotides 694 to 724, and the RNase portion at nucleotides 725 to 1092.

Seq ID No 2 (DNA) gives an exemplary linker encoding sequence that can be arranged betweenVH and VL of an Fv section forming the antigen binding fragment; Seq ID No 3 gives the corresponding amino acid sequence.

Seq ID No 4 gives an exemplary optional signal peptide.

As an example for the conjugate according to the invention, an anti-CD receptor scFvFc-RNase conjugate (I) is presented, the antibody portion being specific for the membrane-bound receptor CD receptor having an extracellular domain, which antibody portion is derived from a human anti-CD receptor antibody. The RNase portion is derived from human pancreatic RNase. In the examples, a variant (II) to the anti-CD receptor scFvFc-RNase conjugate (I) is used, which variant differs in the Fv section only. The similar reactions and properties of these two Fv section variants (I and II) shows that it is the general structure of the conjugates according to the invention that allows for an efficient production in cell culture and provides for the effective antigen specificity and high cytotoxicity.

Example 1: Expression and isolation of anti-CD receptor scFvFc-RNase conjugate

The coding sequence comprised in Seq ID No. 1 was used in nucleic acid constructs, both comprising operably linked in 5' the coding sequence (I and II, respectively) for an Fv portion specific for the CD receptor in the expression vector pCMV myc-ER (obtainable from Invitrogen), which provided operon functions and functional elements for transcription and translation. Expression was transient in HEK293 T-cells.

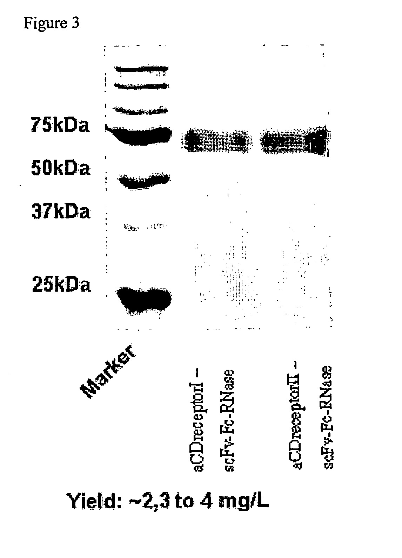

After transfection of HEK293 T-cells, the anti-CD receptor scFvFc-RNase conjugate was isolated from culture supernatants by absorption to a protein A Sepharose column, followed by elution. The yield of pure anti-CD receptor scFvFc-RNase was in the range of 2.3 to 4 mg/L cell culture supernatant. An SDS-PAGE of samples after purification is shown in Figure 3, wherein the conjugate construct and the anti-CD receptor scFv portion variant thereof show about the same molecular weight of approximately 75 kDa.

Size exclusion chromatography of native anti CD receptor scFvFc-RNase was done using 80 mg purified conjugate on a calibrated Superdex 200 10/30 GL column, indicating a molecular weight of approximately 150 kDa, corresponding to a homodimer of native anti-CD receptor scFvFc-RNase with no aggregates being formed.

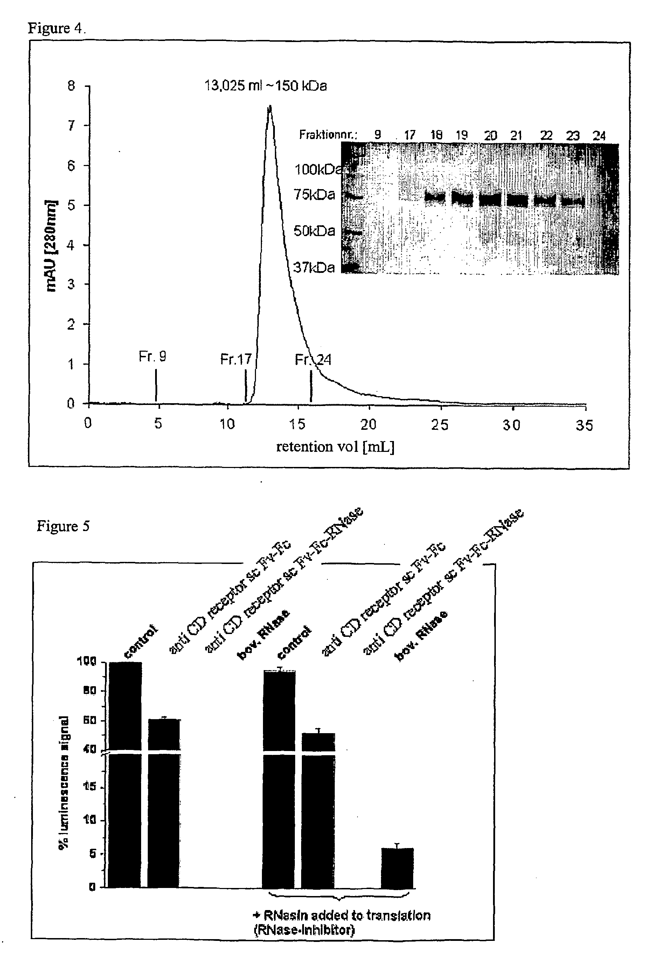

The results of size exclusion chromatography is shown in Figure 4; the inset shows a 10% reducing SDS-PAGE with silver staining of fractions, wherein given fraction numbers are indicated in the SEC chromatogram. According to SDS-PAGE analysis, no impurities are found in the SEC fractions

Example 2: Cell-free RNase activity of anti-CD receptor scFvFc-RNase

In order to assess the RNase activity of anti-CD receptor scFvFc-RNase, Sepharose A - purified preparations were added to in vitro translation reactions of luciferase mRNA using the cell-free translation in rabbit reticulocyte lysate (Promega).

The reduction of luminescence by the presence of RNase activity serves as a direct indicator for the presence of RNase, either from bovine RNase used as a positive control (2.5 µM) or by anti-CD receptor scFvFc-RNase according to the invention (2.5 µM) or using the Ab fragment anti-CD receptor scFvFc (2.5 µM), i.e. without RNase portion as a negative control.

The control for 100% luminescence was without addition of any antibody or RNase preparations. The results, shown in Figure 5, demonstrate that it is only anti-CD receptor scFvFc-RNase according to the invention as well as bovine RNase that provide for an efficient reduction of the luminescence, i.e. degradation of luciferase mRNA in the in vitro translation assay.

Interestingly, the addition of the RNase inhibitor RNasin (obtainable from Promega) to the reaction mixtures resulted in a slight increase in luminescence from both control and antibody preparations (anti-CD receptor scFvFc) as well as an expe cted rise in luminescence in the positive control containing bovine RNase, whereas the anti-CD receptor scFvFc-RNase according to the invention was not impaired in its activity by the RNase inhibitor. RNasin inhibits the activity of RNases, including human pancreatic RNase. Accordingly, RNase activity causing the cytotoxicity of the scFvFc-RNase conjugates is essentially not impaired by RNase inhibitors, exemplified here by RNasin, indicating a stable and efficient cytotoxicity of the conjugates.

Example 3: The binding of anti-CD receptor scFvFc-RNase to CD receptor+ lymphoma

As an example for the antigen-specific binding of conjugates according to the invention, the binding of anti-CD receptor scFvFc-RNase conjugates to lymphoma cells expressing the surface marker CD receptor was assayed by surface plasmon resonance (SPR). Surface plasmon resonance was assayed for both anti-CD receptor variants of anti-CD receptor scFvFc-RNase, using varying concentrations of conjugates on a CM5 chip equipped with immobilized recombinant CD receptor-Fc. As a control, lysozyme was immobilized in a parallel flow cell.

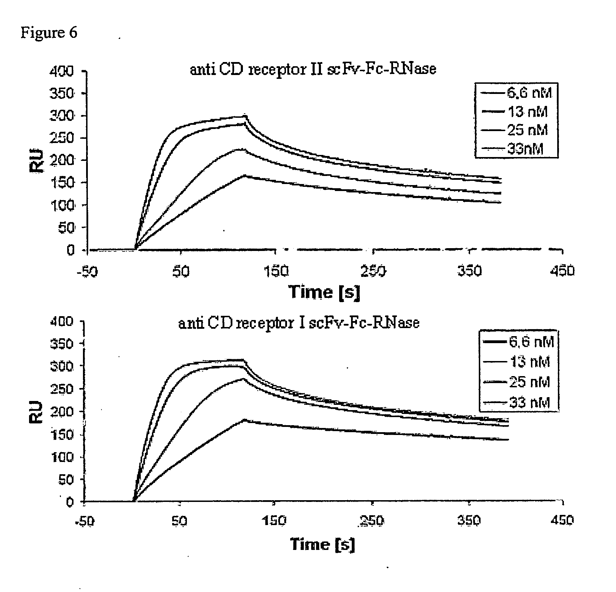

Results are depicted in Figure 6 in relative units (RU). The affinities for the conjugate was determined to approximately 1 nM, which is contrasted by an affinity of approximately 600 nM, determined for a control construct forming a monovalent FcFv-RNase fusion protein.

Example 4: Binding of anti-CD receptor scFvFc-RNase to CD receptor+ cells.

As an example for the binding of conjugates according to the invention to their target cells, i.e cells exhibiting the specific antigen, anti-CD receptor scFvFc-RNase in both variants (I and II) after purification were incubated with CD receptor+ lymphoma cells. As a negative control, CD receptor negative H-DM-YZ cells were used. Staining was done with 10 µg/mL anti-CD receptor scFvFc-RNase conjugates, followed by an anti-human Fc-specific IgG - FITC conjugate as a detection antibody. As an isotype control, Mucin 1 specific IgG was used.

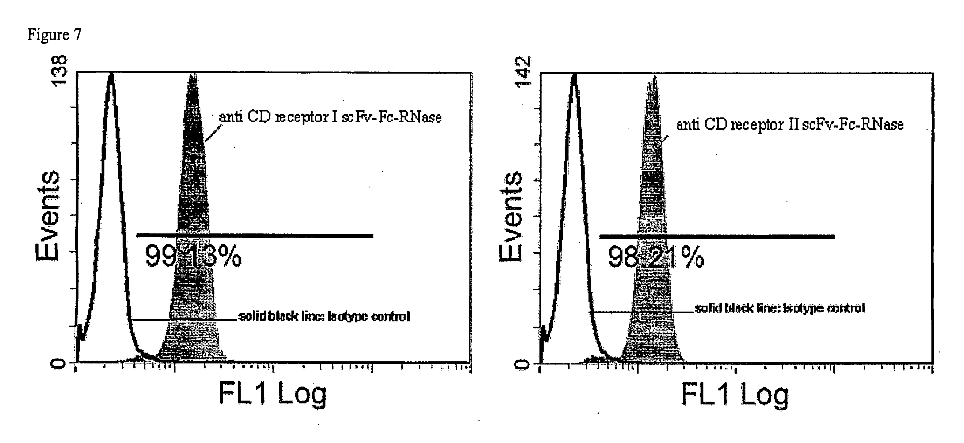

The results of FACS analysis are shown in Figure 7, wherein the peaks for conjugates according to the invention are labelled and the isotype control Mucin 1 specific IgG is depicted by the solid black line. This FACS analysis demonstrates that selective binding of sc Ab-RNase conjugates according to the invention to target cells having the specific antigen is obtained.

Example 5: Internalization of anti-CD receptor cFv RNase by target cells

As an example for an sc Ab-RNase conjugate according to the invention, anti-CD receptor scFvFc-RNase conjugate was incubated with CD receptor+ lymphoma cells at 25 µg/µL. After an incubation for 4 hours at 37 or 4 °C, respectively, CD receptor+ lymphoma cells were analyzed by confocal microscopy following fixation, detecting the scFvFc-RNase conjugate with an anti-human Fc-specific IgG - FITC conjugate and staining of the nucleus with DAPI.

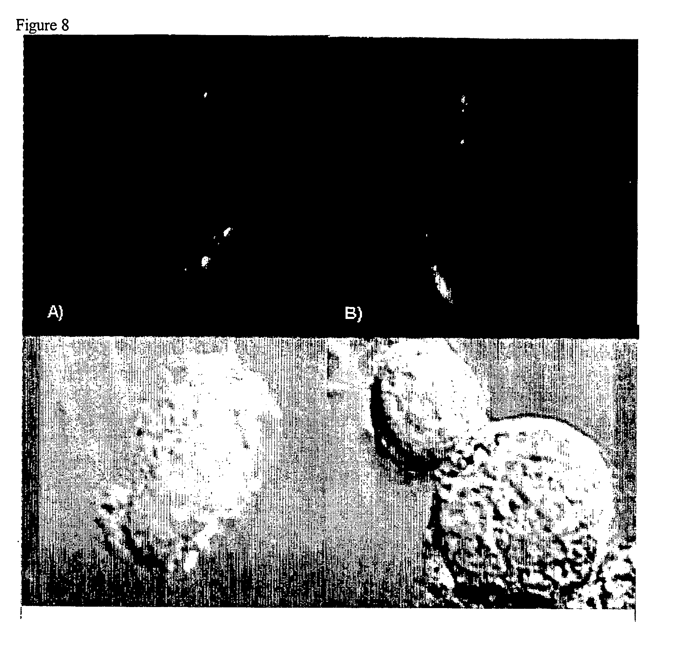

In Figure 8 A, the result of the incubation at 37 °C is shown, in Figure 8 B the incubation 4 °C, with the lower pictures showing overlays with brightfield. These observations demonstrate that conjugates according to the invention are localized within the nucleus, as indicated by the presence of the FITC-labelled detection antibody. At 37 °C, intracellular vesicles are visible in CD receptor+ lymphoma cells (Figure 8 A) containing the conjugates according to the invention, whereas at 4 °C (Figure 8 B), membrane associated localization can be detected. In controls using CD receptor- H-DM-YZ cells, no association of conjugates according to the invention was detected.

Using the example of CD receptor+ lymphoma cells, these results show the antigen-specific detection of target cells and the target cell-specific internalization of sc Ab- . RNase conjugates according to the invention.

Example 6: Cytotoxicity of anti-CD receptor scFvFc-RNase in vitro

As an assay for antigen-specific cytotoxicity of sc Ab-RNase conjugates according to the invention, CD receptor+ lymphoma cells were seeded to 104 cells per well in a 96-well plate in triplicate and incubated for three days with varying concentrations of anti-CD receptor scFvFc-RNase in a range from 0 to 100 nM. The antibody fragment anti-CD receptor scFvFc, i.e. without RNase portion, was included as a negative control.

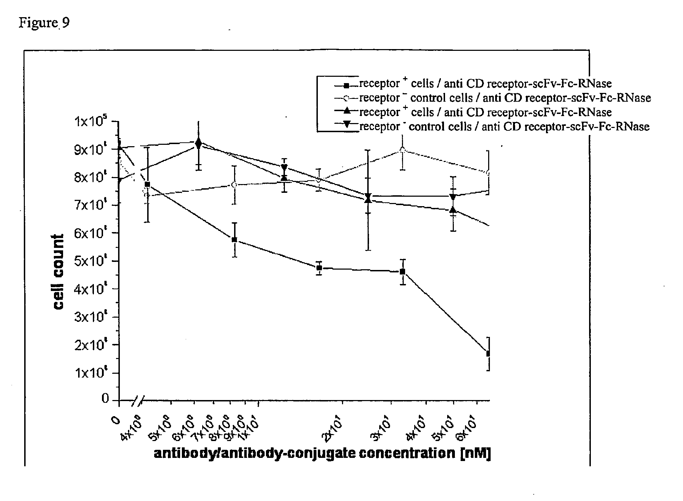

After 3 days incubation at 37°C, the cell number was determined by staining dead cells with trypan blue and counting living cells. Results are shown in Figure 9, demonstrating that it is only the sc Ab-RNase conjugate according to the invention, which strongly inhibits proliferation. For this inhibition, an IC50 of 3.3 nM could be determined, whereas the proliferation of non-target control cells, namely CD receptor- H-DM-YZ cells is not affected.

The negative control using the antibody fragment without RNase portion proves that the inhibition of proliferation is due to the RNase portion of the conjugate according to the invention.

These results could be confirmed by FACS analysis using Annexin V and PI staining, showing the induction of apoptosis and necrosis in tumor cells.

### Sequence List Text

N1037EP.ST25.txt
## Claims

1. Ab-RNase conjugate comprising an antibody (Ab) portion and an RNase (RNase) portion in a single chain (sc) peptide chain,
**characterized in that**
the protein comprises from N-terminus to C-terminus the antibody portion and the RNase portion in the following sections: Fv- hinge - C_{H2} - C_{H3} - linker - RNase, wherein the Fv is V_{H} - linker - V_{L} or V_{L} - linker - V_{H}.

2. Ab-RNase conjugate according to claim 1, **characterized in that** it comprises a signal peptide at its N-terminus.

3. Ab-RNase conjugate according to claim 1 or claim 2, **characterized in that** it forms a dimer upon expression in cell culture.

4. Ab-RNase conjugate according to claim 1 or claim 2, **characterized in that** the RNase portion is selected from the group comprising human pancreatic RNase, angiogenin ribonuclease, bovine seminal RNase, Onconase, and eosinophil derived neurotoxin (EDN).

5. Ab-RNase conjugate according to one of the preceding claims for use as a medicament suitable for the treatment of a neoplastic disease.

6. Ab-RNase conjugate according to one of the preceding claims for use as a medicament suitable for the treatment of parasitic infections.

7. Ab-RNase conjugate according to claim 6 **characterized in that** the parasitic disease is sleeping sickness.

8. Process for producing a single chain Ab-RNase conjugate in cell culture, **characterized in that** the Ab-RNase conjugate comprises from N-terminus to C-terminus an antibody portion and an RNase portion in the following sections:
Fv- hinge - C_{H2} - C_{H3} - linker - RNase, wherein the Fv is V_{H} - linker - V_{L} or V_{L} - linker - V_{H}.

## Patentansprüche

1. Ak-RNase-Konjugat, das einen Antikörper (Ak) -anteil und einen RNase (RNase) - anteil in einer einkettigen (sc) Peptidkette umfasst, **dadurch gekennzeichnet, dass** das Protein vom N-Terminus zum C-Terminus den Antikörperanteil und den RNaseanteil in den folgenden Abschnitten umfasst: Fv- Scharnier - C_{H2} - C_{H3} - Verbinder - RNase, wobei der Fv V_{H} - Verbinder - V_{L} der V_{L} - Verbinder - V_{H} ist.

2. Ak-RNase-Konjugat nach Anspruch 1, **dadurch gekennzeichnet, dass** es an seinem N-Terminus ein Signalpeptid umfasst.

3. Ak-RNase-Konjugat nach Anspruch 1 oder Anspruch 2, **dadurch gekennzeichnet, dass** es bei Expression in Zellkultur ein Dimer bildet.

4. Ak - RNase-Konjugat nach Anspruch 1 oder Anspruch 2, **dadurch gekennzeichnet, dass** der RNaseanteil aus der Gruppe ausgewählt ist, die menschliche Pancreas - RNase, Angiogenin - Ribonuklease, seminale Rinder - RNase, Onkonase und von eosinophilen Leukozyten abgeleitetes Neurotoxin (EDN) umfasst.

5. Ak-RNase-Konjugat nach einem der voranstehenden Ansprüche zur Verwendung als ein Medikament, das zur Behandlung eine neoplastischen Krankheit geeignet ist.

6. Ak-RNase-Konjugat nach einem der voranstehenden Ansprüche zur Verwendung als ein Medikament, das zur Behandlung parasitischer Infektionen geeignet ist.

7. Ak-RNase-Konjugat nach Anspruch 6, **dadurch gekennzeichnet, dass** die parasitische Krankheit die Schlafkrankheit ist.

8. Verfahren zur Herstellung eines einkettigen Ak-RNase-Konjugats in der Zellkultur, **dadurch gekennzeichnet, dass** das Ak-RNase-Konjugat vom N-Terminus zum C-Terminus einen Antikörperanteil und einen RNaseanteil in den folgenden Abschnitten umfasst: Fv- Verbinder - C_{H2} - C_{H3} - Verbinder - RNase, wobei der Fv is V_{H} - Verbinder - V_{L} der V_{L} - Verbinder - V_{H} ist.

## Revendications

1. Conjugué Ab-RNase comprenant une partie anticorps (Ab) et une partie RNase (RNase) dans une chaîne peptidique monocaténaire (sc),
**caractérisé en ce que**, de la terminaison N jusqu'à la terminaison C, la protéine comprend la partie anticorps et la partie RNase dans les sections suivantes : Fv - charnière - C_{H2} - C_{H3} - lieur - RNase, Fv étant V_{H} - lieur - V_{L} ou V_{L} - lieur - V_{H}.

2. Conjugué Ab-RNase selon la revendication 1, **caractérisé en ce qu'**il comprend un peptide signal à sa terminaison N.

3. Conjugué Ab-RNase selon la revendication 1 ou la revendication 2, **caractérisé en ce qu'**il forme un dimère quand il est exprimé dans une culture de cellules.

4. Conjugué Ab-RNase selon la revendication 1 ou la revendication 2, **caractérisé en ce que** la partie RNase est choisie parmi le groupe qui comprend la RNase pancréatique de l'homme, la ribonucléase appelée angiogénine, la RNase séminale du bovin, l'Onconase et la neurotoxine dérivée de l'éosinophile (EDN).

5. Conjugué Ab-RNase selon l'une des revendications précédentes à utiliser comme médicament adapté au traitement d'une maladie néoplasique.

6. Conjugué Ab-RNase selon l'une des revendications précédentes à utiliser comme médicament adapté au traitement d'infections parasitaires.

7. Conjugué Ab-RNase selon la revendication 6, **caractérisé en ce que** la maladie parasitaire est la maladie du sommeil.

8. Procédé pour produire un conjugué Ab-RNase monocaténaire dans une culture de cellules, **caractérisé en ce que**, de la terminaison N jusqu'à la terminaison C, le conjugué Ab-RNase comprend une partie anticorps et une partie RNase dans les sections suivantes : Fv- charnière - C_{H2} - C_{H3} - lieur - RNase, Fv étant V_{H} - lieur - V_{L} ou V_{L} - lieur - V_{H}.


SEQUENCE LISTING
<110> Technische Universität Braunschweig
<120> Antibody-RNase-Conjugate
<130> N1037EP
<160> 4
<170> PatentIn version 3.1
<210> 1
   211> 1092
   <212> DNA
   <213> Artificial Sequence
<220>
<223> synthetic antibody-RNase-conjugate partial sequence
<220>
   <221> exon
   <222> (1)..(54)
   <223> hinge
<220>
   <221> exon
   <222> (55)..(385)
   <223> CH2 domain
<220>
   <221> exon
   <222> (386)..(693)
   <223> CH3 domain
<220>
   N1037EP.ST25.txt
<221> exon
   <222> (694)..(724)
   <223> linker
<220>
   <221> exon
   <222> (725)..(1092)
   <223> human pancreatic RNase
<400> 1
<210> 2 <211> 45

   <212> DNA
   <213> Artificial Sequence
<220>
<223> synthetic antibody-RNase-conjugate partial sequence
<220>
   <221> CDS
   <222> (1)..(45)
   <223> Gly-Ser linker
<400> 2
   N1037EP.ST25.txt
<210> 3
   <211> 15
   <212> PRT
   <213> Artificial Sequence
<220>
<223> synthetic antibody-RNase-conjugate partial sequence
<400> 3
   Gly Gly Gly Gly Ser Gly Gly Gly Gly Ser Gly Gly Gly Gly Ser
   1 5 10 15
<210> 4
   <211> 139
   <212> DNA
   <213> Artificial Sequences
<220>
<223> synthetic antibody-RNase-conjugate partial sequence
<220>
   <221> exon
   <222> (1)..(45)
   <223> signal peptide
<220>
   <221> Intron
   [215] <222> (46)..(127)
   <223>
<220>
   <221> exon
   <222> (128)..(139)
   <223>
   N1037EP.ST25.txt
<400> 4
   atg gga tgg agc tgt atc atc ctc ttc ttg gta gca aca gct aca 45
   Met Gly Trp Ser Cys Ile Ile Leu Phe Leu Val Ala Thr Ala Thr
   1 5 10 15
   ggtaaggggt taacagtagc aggcttgagg tctggacata tatatgggtg acaatgacat 105
   ccactttgcc tttctctcca ca ggc gcg cac tcc 139
   Gly Ala His Ser